# FASCICULE DE BREVET EUROPEEN

(11) **EP 1 621 245 B1**
(45) Date de publication et mention de la délivrance du brevet: **13.10.2010**
(21) Numéro de dépôt: 05291603.8
(22) Date de dépôt: 27.07.2005
(51) Int. Cl.: B01D 53/04, B01D 53/00, A61L 9/16

(54) **Procédé de traitement de composés organiques volatils ou gaz, notamment pour un conduit de circulation d'un flux d'air**
Verfahren zur Behandlung von flüchtigen organischen Stoffe oder Gase, insbesondere in einem Luftströmungsrohr
Process for treating volatile organic compounds or gases, especially in an air circulation duct

(30) Priorité: 30.07.2004 FR 0408469
(43) Date de publication de la demande: 01.02.2006
(73) Titulaire: FAGORBRANDT SAS, 92500 Rueil Malmaison (FR)
(72) Inventeur: Chevrier, Jean-Paul, 45590 St Cyr En Val (FR); Bertrand, Cyrille, 45430 Checy (FR); Philibert, Emmanuel, 45000 Orleans (FR)
(74) Mandataire: Stankoff, Hélène

(56) Documents cités:
- EP-A- 0 798 143
- US-A- 5 145 648
- US-B1- 6 358 374
- PATENT ABSTRACTS OF JAPAN vol. 012, no. 005 (C-467), 8 janvier 1988 (1988-01-08) & JP 62 163730 A (EBARA INFILCO CO LTD; others: 01), 20 juillet 1987 (1987-07-20)

## Description

La présente invention concerne un conduit de circulation d'un flux d'air comprenant un dispositif de traitement de composés organiques volatils ou gaz dans un flux d'air, ainsi qu'une hotte d'aspiration.

De manière générale, la présente invention concerne le traitement des odeurs ou des gaz, notamment dans un flux d'air mis en circulation.

Un tel procédé peut être notamment mis en oeuvre dans une hotte d'aspiration utilisée en recirculation, c'est-à-dire que l'air est réintroduit dans le milieu ambiant après traitement. Il est alors nécessaire de capturer les molécules odorantes (composés organiques volatils ou gaz) transportées par le flux d'air au moyen d'un filtre placé sur le parcours du flux d'air dans la hotte d'aspiration associée à un conduit de circulation du flux d'air.

Traditionnellement, on dispose un filtre à base de charbon actif au travers duquel un flux d'air est aspiré grâce à des moyens d'aspiration, tels qu'un ventilateur.

Le charbon actif est en effet un excellent adsorbant, permettant de capturer des composés organiques volatils ou gaz contenus dans le flux d'air.

Un filtre à base de charbon actif se présente généralement sous la forme d'un filtre en mousse avec dépôt de charbon actif en poudre sur la mousse, ou encore sous la forme d'éléments de charbon actif enfermés dans des sachets réalisés en un matériau perméable au flux d'air à traiter.

L'inconvénient majeur de ces filtres en charbon actif est leur courte durée de vie, le charbon actif étant rapidement saturé et devenant inefficace pour la capture de composés organiques volatils ou gaz.

Traditionnellement, lorsqu'un tel filtre à base de charbon actif est utilisé dans une hotte d'aspiration domestique, son efficacité se limite à quelques mois, alors que la durée de vie d'une hotte est de l'ordre d'une dizaine d'années.

L'utilisateur doit alors changer le filtre à base de charbon actif, ce qu'il fait dans moins de 10% des cas d'emplois.

On connaît un conduit de circulation d'un flux d'air tel que décrit dans le document US 5 145 648 qui comprend des moyens d'aspiration et un filtre à base de charbon actif.

Le filtre en charbon actif est associé à des moyens de chauffage et les moyens d'aspiration sont adaptés à aspirer le flux d'air selon au moins deux débits d'aspiration différents.

Des moyens de destruction par oxydation catalytique sont mis en oeuvre.

Toutefois, ces moyens de destruction requièrent la présence d'une source de chaleur.

La présente invention propose un conduit de circulation d'un flux d'air dans lequel le filtre à base de charbon actif est disposé en amont d'un matériau photocatalytique activable par des moyens formant source de lumière relativement au sens de circulation du flux d'air dans le conduit de circulation, le matériau photocatalytique et les moyens formant source de lumière étant intégrés dans un conduit auxiliaire de circulation du flux d'air, des moyens de dérivation du flux d'air associés aux moyens d'aspiration étant adaptés à diriger le flux d'air soit dans le conduit de circulation, soit uniquement dans le conduit auxiliaire.

L'objet de la présente invention est défini dans la revendication 1.

Selon une caractéristique avantageuse de l'invention, le filtre en charbon actif est chauffé à une température comprise entre 40°C et 120°C.

L'échauffement du charbon actif provoque la libération des composés organiques volatils ou gaz emprisonnés dans le charbon actif, permettant ainsi la désorption du filtre.

La disposition du filtre relativement au matériau photocatalytique dans le conduit de circulation du flux d'air permet de mettre en oeuvre tout d'abord une phase de capture des composés organiques volatils ou gaz dans le filtre à base de charbon actif, puis leur destruction au moyen du matériau photocatalytique activé par les moyens formant source de lumière.

Grâce à la disposition du matériau photocatalytique dans un conduit auxiliaire, celui-ci n'obture pas le conduit de circulation principal, évitant ainsi de créer un dysfonctionnement dans l'aspiration du flux d'air lors de la phase de capture des composés organiques volatils ou gaz dans le filtre à base de charbon actif.

En pratique, les moyens de dérivation comprennent au moins un clapet s'étendant au travers du conduit de circulation et adapté dans une première position à ouvrir le conduit de circulation et dans une seconde position à obturer ledit conduit de circulation hormis ledit conduit auxiliaire.

Ainsi, le flux d'air peut passer alternativement, en fonction de la position du clapet, dans le conduit de circulation principal ou uniquement dans le conduit auxiliaire comportant les moyens formant source de lumière associés au matériau photocatalytique.

De préférence, le ou les clapets sont placés dans la première position sous l'effet de la force exercée par le flux d'air lorsque le débit d'aspiration des moyens d'aspiration est supérieur à une valeur de seuil prédéterminée.

Lorsque le débit d'aspiration du flux d'air est important, ce flux d'air passe dans le conduit de circulation principal, évitant toute perte de charge dans le débit du flux d'air qui serait lié à la présence du matériau photocatalytique et des moyens formant source de lumière.

De préférence, le conduit auxiliaire est réalisé au moyen d'un filtre recouvert d'un matériau photocatalytique.

Ainsi, lors du passage du flux d'air au travers du conduit auxiliaire, l'intégralité du flux d'air vient en contact avec le matériau photocatalytique et peut ainsi transporter et déposer les composés organiques volatils ou gaz à détruire sur le filtre revêtu du matériau photocatalytique.

La présente invention concerne enfin une hotte d'aspiration comprenant un conduit de circulation conforme à l'invention.

Cette hotte d'aspiration présente des avantages et caractéristiques analogues à ceux décrits précédemment.

Grâce à l'invention, cette hotte d'aspiration peut être utilisée en recirculation sans rejet de molécules odorantes dans l'air ambiant, pendant toute sa durée de vie et sans requérir un changement du filtre en charbon actif.

D'autres particularités et avantages de l'invention apparaîtront encore dans la description ci-après.

Aux dessins annexés, donnés à titre d'exemples non limitatifs :
- la figure 1 illustre schématiquement une hotte d'aspiration conforme à un premier mode de réalisation de l'invention, pendant une phase de capture du procédé de traitement ;
- la figure 2 est une vue analogue à la figure 1, pendant une phase de désorption et de destruction des composés organiques volatils ou gaz du procédé de traitement;
- la figure 3 est une vue schématique d'un filtre à charbon selon un mode de réalisation de l'invention ; et
- la figure 4 est une vue en perspective illustrant le montage d'un dispositif de destruction des composés organiques volatiles dans un conduit de hotte d'aspiration conforme au premier mode de réalisation de l'invention.

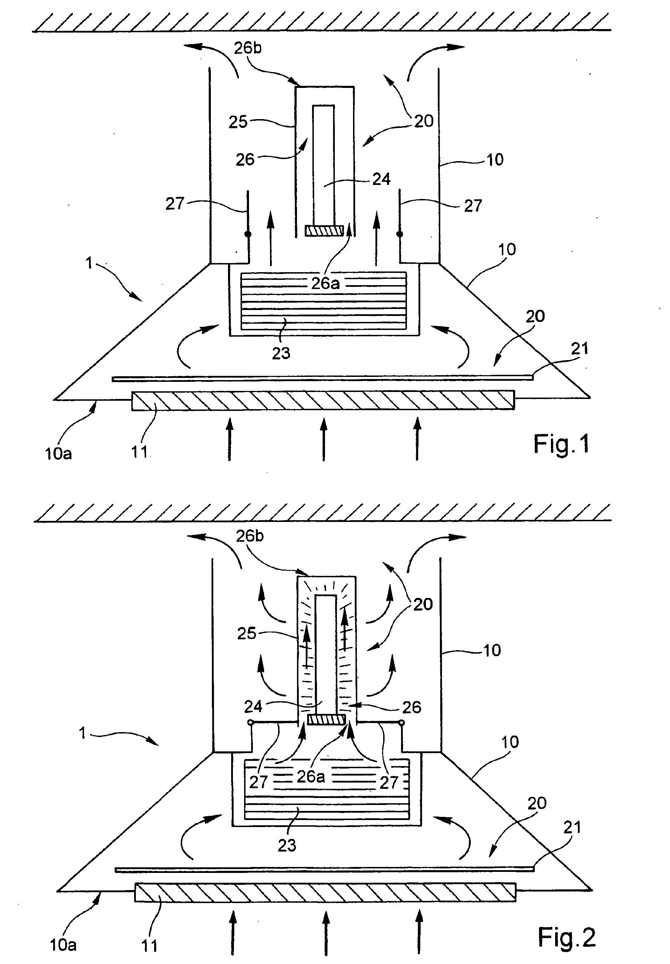

On va décrire tout d'abord en référence à la figure 1, une hotte d'aspiration conforme à un premier mode de réalisation de l'invention.

Une telle hotte d'aspiration peut être à usage domestique et placée au-dessus d'un plan de cuisson afin d'aspirer et traiter les fumées de cuisson.

Le flux d'air, dont la circulation dans la hotte est représentée par les différentes flèches sur la figure 1, est réintroduit dans l'air ambiant.

Cette hotte est constituée principalement d'un conduit de circulation 10 du flux d'air dont un plan d'extrémité 10a disposé en vis-à-vis du plan de cuisson, comporte une ouverture pour le passage du flux d'air. Cette ouverture est traditionnellement obturée par un filtre à graisse 11, par exemple réalisé à partir de métal déployé. Le fonctionnement d'un tel filtre à graisse, permettant de bloquer et de figer les molécules de graisse présentes dans les fumées de cuisson, n'a pas besoin d'être décrit plus en détail ici.

Le conduit de circulation 10 comprend un dispositif de traitement 20 des composés organiques volatils ou gaz adapté à traiter et détruire les molécules odorantes présentes dans le flux d'air avant son rejet dans l'air ambiant.

Comme bien illustré à la figure 1, ce dispositif de traitement 20 comporte plusieurs éléments associés les uns avec les autres.

Il comporte tout d'abord un filtre à base de charbon actif 21.

Ce filtre à base de charbon actif peut être fixé sur les parois latérales du conduit de circulation 10. Ce filtre à base de charbon actif 21 est de préférence associé à des moyens de chauffage.

Pour cela le filtre en charbon actif 21 peut être réalisé en une toile de charbon actif conductrice. Cette toile de charbon actif est adaptée à être connectée électriquement à deux de ses extrémités, de telle façon que lors du passage d'un courant électrique dans la toile de charbon actif, celle-ci puisse s'échauffer par effet Joule.

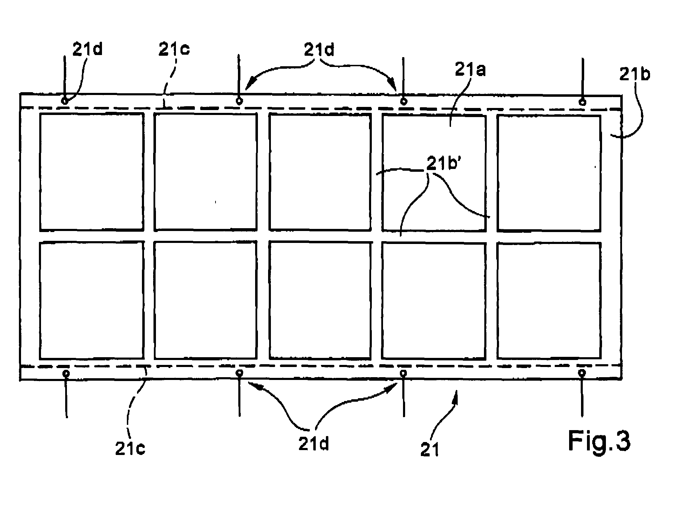

Un exemple d'un filtre en charbon actif 21 est illustré notamment à la figure 3.

La toile de charbon actif 21 a peut être réalisée à partir d'un tissu ou de deux mats de verre entre lesquels sont disposés des grains ou poudre de charbon actif.

Le charbon actif est conducteur de telle sorte qu'un courant électrique peut traverser la toile de charbon actif.

De préférence, pour assurer le maintien de cette toile en charbon actif 21 a lors du passage du flux d'air, un cadre 21 b est placé autour de la toile de charbon actif 21 a. Dans cet exemple de réalisation, le cadre 21 b comprend en outre des nervures 21 b' montées en croisillons dans le plan de la toile de charbon actif 21 a afin de maintenir celle-ci dans un plan.

Les connexions électriques sont réalisées sur deux bords opposés de la toile de charbon actif 21 a.

Deux portions de ruban en cuivre 21c sont situés respectivement, au niveau des bords opposés de la toile de charbon actif 21 a, sous le cadre 21 b.

Des connexions électriques sont réalisées à l'aide de conducteurs 21d soudés sur les rubans en cuivre, à l'aide d'une colle conductrice.

La connexion électrique pourrait être réalisée différemment, par exemple à l'aide d'un adhésif métallique conducteur du type adhésif en aluminium, pénétrant dans le filtre.

Tout autre assemblage mécanique, par exemple pour former un contact électrique par pression au niveau du cadre 21 b, pourrait également être envisagé.

En outre, la toile de charbon actif 21 a pourrait également être réalisée à partir d'un tissu en carbone. Une toile de carbone peut être rendue conductrice grâce à un taux de carbonisation très élevé, de l'ordre de 96 à 98%.

Le charbon actif peut ainsi être échauffé dans un tel mode de réalisation, à une température homogène sur toute sa surface.

Par ailleurs, un asservissement en température à l'aide d'un capteur de température positionné en contact avec la toile de charbon actif peut être adapté à réguler en température l'échauffement du filtre à charbon actif.

Comme cela sera décrit ultérieurement en référence au procédé de traitement, le chauffage du charbon actif permet la désorption de ce filtre. En fonction du type de charbon actif utilisé, la température de désorption peut être comprise entre 40°C et 120°C et de préférence entre 60 et 120°C, pour permettre la libération des composés organiques volatils ou gaz capturés dans le charbon actif. Cette température d'échauffement est de préférence égale environ à 80°C.

Bien entendu, d'autres types de moyens de chauffage du filtre en charbon actif pourraient être utilisés.

Par exemple, le charbon actif peut être chauffé par convection, au moyen du passage d'un flux d'air chaud, préchauffé à l'aide d'un élément chauffant du type d'une résistance électrique placée en amont du filtre en charbon actif relativement au sens de circulation du flux d'air.

L'échauffement du charbon actif peut également être obtenue par conduction, en plaçant un élément chauffant du type d'une résistance électrique directement en contact avec le filtre en charbon actif.

Dans ces autres modes de réalisation de chauffage du filtre en charbon actif, celui-ci peut être réalisé de façon classique à l'aide d'une structure plane en mousse comportant un dépôt de charbon actif en poudre, ou encore au moyen d'élément de charbon actif emprisonné dans des sachets.

Outre le filtre à charbon actif 21 et les moyens de chauffage associés, le dispositif de traitement du flux d'air comprend également des moyens d'aspiration 23. Ces moyens d'aspiration 23 peuvent être constitués de manière classique par un ventilateur adapté à aspirer un flux d'air à travers le conduit de circulation 10.

Ces moyens d'aspiration 23 sont adaptés à aspirer le flux d'air selon au moins deux débits d'aspiration différents.

Comme cela sera expliqué clairement en relation avec le procédé de traitement conforme à l'invention, un premier débit d'aspiration peut être compris entre 150 et 1200 m³/h. Ce débit relativement élevé d'aspiration est bien adapté à aspirer et à évacuer l'ensemble des fumées de cuisson s'élevant au niveau de la hotte d'aspiration.

De manière classique, la hotte d'aspiration peut comporter des moyens de réglages accessibles par l'utilisateur permettant de régler le débit d'aspiration des moyens de ventilation 23 à différentes valeurs comprises entre 150 et 1200 m³/h, et par exemple selon quatre niveaux de débit égals à 150 m³/h, 400 m³/h, 800 m³/h et 1200 m³/h.

Les moyens de ventilation 23 peuvent également être adaptés à aspirer un flux d'air selon un second débit, bien inférieur au premier débit, compris par exemple entre 10 et 50 m³/h, de préférence entre 20 et 40 m³/h, et de préférence égal à 30 m³/h.

Le dispositif de traitement 20 comporte en outre un matériau photocatalytique activable par des moyens formant source de lumière 24.

Dans cet exemple de réalisation, le matériau photocatalytique est un dioxyde de titane (TIO₂) déposé sur un filtre papier 25.

Les moyens formant source de lumière 24 sont réalisés à partir d'une ou plusieurs sources lumineuses UVA dont le rayonnement émet avec une longueur d'onde d'environ 365 nanomètres plus ou moins 20 nanomètres.

Ces sources lumineuses peuvent être des lampes fluorescentes ou encore des diodes électroluminescentes (LEDs) émettant dans l'UVA.

Sous l'effet de cette irradiation d'UVA, le dioxyde de titane est activé pour détruire par réaction catalytique les composés organiques volatils ou gaz transportés par un flux d'air venant en contact avec et traversant le filtre revêtu du matériau photocatalytique 25.

Comme illustré à la figure 1, dans ce mode de réalisation, le filtre à base de charbon actif 21 est disposé en amont du matériau photocatalytique 25 et de la source lumineuse 24 relativement au sens de circulation du flux d'air dans le conduit de circulation 10, illustré par les flèches sur la figure 1.

Plus particulièrement, le filtre à base de charbon actif est disposé à proximité du filtre à graisse 11, sensiblement au niveau de l'ouverture d'aspiration de la hotte 1.

Le filtre recouvert de matériau photocatalytique 25 et la source de lumière 24 sont disposés en aval dans le conduit 10.

Les moyens de ventilation 23 sont disposés entre le filtre à charbon actif 21 et le filtre revêtu d'un matériau photocatalytique 25.

Bien entendu, les moyens de ventilation 23 pourraient être disposés en aval du filtre revêtu d'un matériau photocatalytique 25.

Le matériau photocatalytique déposé sur le filtre 25 et les moyens formant source de lumière 24 sont intégrés dans un conduit auxiliaire de circulation du flux d'air. Des moyens de dérivation, comprenant des clapets 27 sont adaptés à diriger le flux d'air soit dans le conduit de circulation 10, soit uniquement dans le conduit auxiliaire 26.

Dans le mode de réalisation illustré sur les figures 1 et 2, le conduit auxiliaire 26 est réalisé directement au moyen du filtre 25 recouvert du matériau photocatalytique.

Ce filtre 25 est conformé sous la forme d'un conduit ouvert à l'une de ses extrémités 26a, au niveau de l'introduction du flux d'air et fermé à son extrémité opposée 26b.

Le conduit auxiliaire 26 s'étend ainsi dans une direction longitudinale parallèle à la direction longitudinale du conduit principal 10 de telle sorte que son extrémité ouverte 26a est placée en amont de son extrémité fermée 26b relativement au sens de circulation du flux d'air.

Le flux d'air peut être introduit dans ce conduit auxiliaire 26 et en sortir en traversant le filtre 25 revêtu du matériau photocatalytique.

Bien entendu, d'autres modes de réalisation d'un tel conduit auxiliaire pourraient être envisagés. En particulier, ce conduit auxiliaire pourrait être réalisé à partir d'une plaque de métal ou de plastique fixée sur l'une des parois intérieures du conduit principal 10, un filtre recouvert de matériau photocatalytique étant par exemple placé en travers de ce conduit auxiliaire de manière à être traversé par le flux d'air à traiter.

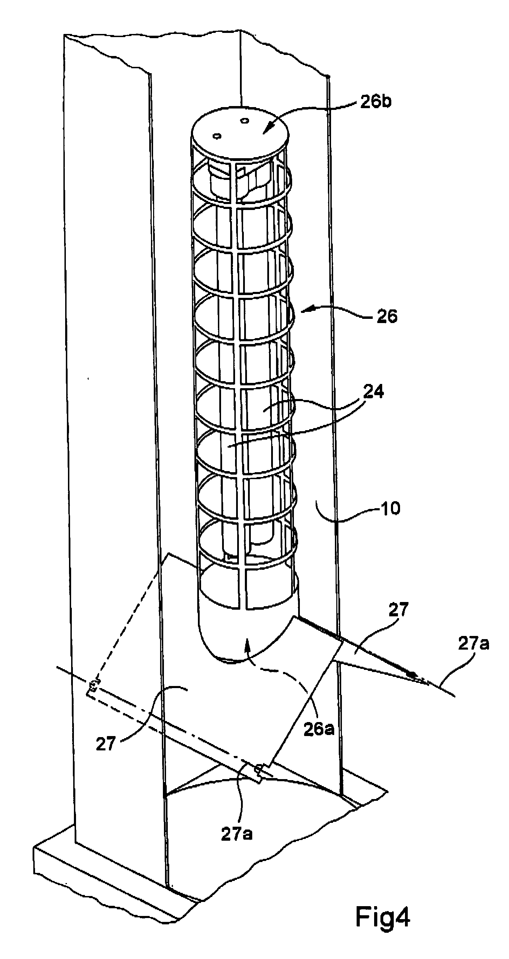

On a illustré à titre d'exemple à la figure 4 une réalisation pratique d'un conduit auxiliaire 26.

Dans cet exemple, le conduit auxiliaire est réalisé à partir d'un support grillagé fixé par des moyens de fixation (non représentés) au conduit principal 10 de telle sorte que son extrémité ouverte 26a est placée en amont de son extrémité fermée 26b relativement au sens de circulation du flux d'air.

Ce conduit auxiliaire grillagé 26 laisse ainsi passer le flux d'air. Ses parois sont recouvertes d'un filtre papier (oté sur la figure 5) au travers duquel le flux d'air est aspiré.

Dans cet exemple de réalisation, une source lumineuse UVA 24 est fixée au conduit auxiliaire 26, au niveau de son extrémité aval fermée 26b.

Des clapets 27 de dérivation du flux d'air sont montés sur le conduit principal 10 de part et d'autre du conduit auxiliaire 26.

Les clapets 27 de dérivation du flux d'air sont mobiles autour d'un axe de pivotement 27a entre deux positions.

Dans une première position, telle qu'illustrée à la figure 1, les clapets sont adaptés à ouvrir le conduit de circulation 10 de telle sorte que le flux d'air peut circuler dans le conduit principal 10 ainsi que dans le conduit auxiliaire 26.

Dans une seconde position, telle qu'illustrée à la figure 2 et à la figure 4, les clapets 27 sont adaptés à obturer le conduit de circulation 10 hormis le conduit auxiliaire 26.

Dans cette position, le flux d'air passe uniquement au travers du conduit auxiliaire 26 de telle sorte que l'intégralité du flux d'air traverse le filtre revêtu d'un matériau photocatalytique 25.

De préférence, les clapets 27 sont mobiles automatiquement, sous l'effet de la force exercée par le flux d'air aspiré dans le conduit de circulation 10.

Dans un tel cas, les clapets 27 sont maintenus au repos dans leur seconde position telle qu'illustrée à la figure 2.

Lorsque le débit d'aspiration des moyens d'aspiration 23 est supérieur à une valeur de seuil prédéterminée, le flux d'air est adapté à déplacer les clapets 27 dans la première position telle qu'illustrée à la figure 1.

La valeur de seuil prédéterminée peut correspondre par exemple à la valeur basse du premier débit d'aspiration des moyens d'aspiration 23, de l'ordre de 150 m³/h.

Lorsque le débit d'aspiration devient inférieur à cette valeur, les clapets 27 peuvent être rappelés par des moyens de rappel élastiques dans leur seconde position telle qu'illustrée à la figure 2.

Bien entendu, ces clapets 27 pourraient être déplacés par tout type de commande électromécanique, par exemple associée à la valeur du débit d'aspiration des moyens d'aspiration.

On va décrire à présent en référence au mode de réalisation illustré aux figures 1 et 2, un procédé de traitement des composés organiques volatils ou gaz conforme à l'invention.

Lorsque la hotte telle qu'illustrée à la figure 1 est placée au dessus d'un plan de cuisson, le procédé de traitement a pour but de détruire les composés organiques volatils ou gaz générateur d'odeurs présents dans le flux d'air aspiré par les moyens d'aspiration 23.

Ce procédé de traitement comporte différentes phases.

Il comporte tout d'abord une phase de capture des composés organiques volatils ou gaz contenus dans le flux d'air au niveau du charbon actif du filtre 21.

Dans cette phase de capture, le flux d'air, aspiré par les moyens d'aspiration 23, après avoir traversé le filtre à graisse 11, traverse le filtre à charbon actif 21.

Le charbon actif est un très bon adsorbant de telle sorte qu'il est parfaitement adapté à capturer les composés organiques volatils ou gaz au niveau des particules de charbon actif. Le flux d'air, après avoir traversé le filtre à charbon actif, est entraîné dans le conduit principal 10 et est ensuite rejeté dans l'air ambiant.

Dans cette phase de capture des composés organiques volatils ou gaz au niveau du filtre à charbon actif 21, le flux d'air est aspiré selon un premier débit d'aspiration des moyens d'aspiration 23 compris entre 150 et 1200 m³/h.

Comme décrit précédemment, ce premier débit d'aspiration peut être généralement réglé par l'utilisateur en fonction de la quantité de fumée à évacuer au niveau du plan de cuisson.

Sous l'effet de ce premier débit d'aspiration, supérieur à la valeur de seuil prédéterminée de l'ordre de 150 m³/h, les clapets 27 sont dans une première position ouverte telle qu'illustrée à la figure 1 de telle sorte que l'ensemble du flux d'air circule à l'intérieur du conduit de circulation 10, sans perte de charge lié à la présence du système de destruction d'odeur constitué par le filtre revêtu du matériau photocatalytique 25 et de la source lumineuse associée 24.

Le filtre à charbon actif 21 remplit ainsi parfaitement sa fonction de traitement des composés organiques volatils ou gaz, en piégeant ceux-ci à l'intérieur du filtre, tant qu'il n'est pas saturé.

Pour conserver son efficacité, le procédé de traitement comporte ensuite une phase de désorption du charbon actif permettant de retirer les composés organiques volatils ou gaz prisonniers du charbon actif.

Cette phase de désorption du charbon actif est mise en oeuvre en chauffant le filtre de charbon actif 21, par exemple grâce à un auto-échauffement de celui-ci par passage d'un courant électrique au travers de la toile de charbon actif telle que décrite précédemment.

L'échauffement du charbon actif à une température comprise entre 40 et 120°C provoque la libération des composés organiques volatils ou gaz capturés dans le filtre 21.

Afin d'entraîner hors du filtre à charbon actif 21 les composés organiques volatils ou gaz ainsi libérés, les moyens d'aspiration 23 sont adaptés à aspirer un flux d'air selon un second débit d'aspiration inférieur au premier débit d'aspiration décrit précédemment.

Ce second débit d'aspiration est compris entre 10 et 50 m³/h, et est de préférence égal à 30 m³/h.

Le flux d'air entraîné à ce second débit d'aspiration traverse ainsi le filtre à charbon actif 21 et entraîne les composés organiques volatils ou gaz libérés par le chauffage de ce filtre.

Le faible débit du flux d'air ne provoque pas l'ouverture des clapets 27 de telle sorte que, comme illustré à la figure 2, l'intégralité du flux d'air aspiré selon ce second débit d'aspiration est introduit dans le conduit auxiliaire 26.

Les composés organiques volatils ou gaz ainsi véhiculés dans le flux d'air sont aspirés au travers du filtre 25 et viennent ainsi en contact du matériau photocatalytique. Lorsque celui-ci est activé par la source lumineuse 24, les composés organiques volatils ou gaz sont détruits par photocatalyse au niveau du filtre 25.

De préférence, afin d'éviter tout rejet d'un flux d'air chargé d'odeurs dans l'air ambiant, la phase de désorption du charbon actif et la phase de destruction des composés organiques volatils ou gaz sont mises en oeuvre simultanément.

Ainsi, en pratique, le chauffage du filtre à charbon actif 21 est mis en oeuvre simultanément à l'allumage de la source de lumière 24 permettant l'activation du matériau photocatalytique.

Grâce au faible débit, de l'ordre de 30 m³/h, des moyens d'aspiration pendant ces phases de désorption et de destruction des composés organiques volatils ou gaz, le bruit de la hotte 1 est imperceptible par l'utilisateur.

Ainsi, il est avantageux de prévoir que les phases de désorption et de destruction soient mises en oeuvre en continu, en dehors des périodes de fonctionnement de la hotte pendant lesquelles la phase de capture dans le filtre de charbon actif est mise en oeuvre.

À titre de variante, la mise en oeuvre des phases de désorption et de destruction pourrait être commandée automatiquement grâce à une mesure donnée par un capteur d'odeur placé en aval du filtre en charbon actif.

Ce capteur d'odeur pourrait indiquer la saturation du filtre en charbon actif et ainsi commander la mise en oeuvre des phases de désorption et de destruction dès que le filtre à charbon actif est saturé.

La mise en oeuvre des phases de désorption et de destruction pourrait également être réalisée sur la base d'informations diverses relatives au fonctionnement de la hotte, et par exemple lorsqu'une durée de fonctionnement de la hotte ou un nombre de mises en marche de cette hotte a atteint une valeur seuil prédéterminée.

Tout autre type de fonctionnement permettant l'alternance de la phase de capture dans le filtre de charbon actif des molécules odorantes et des phases de désorption et de destruction de ces molécules odorantes peut être envisagé.

Grâce à ce procédé de traitement, le filtre de charbon actif peut être désorbé de telle sorte qu'il n'est jamais saturé. Il n'est pas nécessaire de changer celui-ci pendant toute la durée de vie de la hotte.

## Revendications

1. Conduit de circulation d'un flux d'air comprenant un dispositif de traitement de composés organiques volatils ou gaz dans un flux d'air comprenant des moyens d'aspiration (23) et un filtre à base de charbon actif (21, 31), le filtre en charbon actif (21, 31) étant associé à des moyens de chauffage et les moyens d'aspiration (23) étant adaptés à aspirer le flux d'air selon au moins deux débits d'aspiration différents, ledit dispositif de traitement comprenant un conduit auxiliaire (26) de circulation du flux d'air intégrant un matériau photocatalytique et des moyens formant source de lumière (24), le filtre à base de charbon actif (21, 31) étant disposé en amont du matériau photocatalytique activable par les moyens formant source de lumière (24, 32) relativement au sens de circulation du flux d'air dans le conduit de circulation (10), le conduit de circulation comprenant en outre des moyens de dérivation (27) du flux d'air comprenant au moins un clapet adapté dans une première position à diriger le flux d'air dans le conduit de circulation (10) et dans une seconde position à diriger le flux d'air uniquement dans le conduit auxiliaire (26), ledit au moins un clapet (27) étant placé dans ladite première position lorsque le débit d'aspiration des moyens d'aspiration (23) est supérieur à une valeur de seuil prédéterminée.

2. Conduit de circulation conforme à la revendication 1, **caractérisé en ce que** ledit au moins un clapet (27) s'étend au travers du conduit de circulation (10) et est adapté dans ladite première position à ouvrir le conduit de circulation (10) et dans ladite seconde position à obturer ledit conduit de circulation (10) hormis ledit conduit auxiliaire (26).

3. Conduit de circulation conforme à la revendication 2, **caractérisé en ce que** ledit au moins un clapet (27) est placé dans ladite première position sous l'effet de la force exercée par le flux d'air.

4. Conduit de circulation conforme à l'une des revendications 1 à 3, **caractérisé en ce qu'**un premier débit d'aspiration est compris entre 150 et 1200m³/h et un second débit d'aspiration est compris entre 10 et 50 m³/h.

5. Conduit de circulation conforme à l'une des revendications 1 à 4, **caractérisé en ce que** le conduit auxiliaire (26) est réalisé au moyen d'un filtre (25) recouvert d'un matériau photocatalytique.

6. Conduit de circulation conforme à l'une des revendications 1 à 5, **caractérisé en ce que** le filtre en charbon actif (21, 31) est réalisé en une toile de charbon actif adaptée à être connectée électriquement à deux extrémités de façon à s'échauffer par effet Joule.

7. Conduit de circulation conforme à l'une des revendications 1 à 6, **caractérisé en ce que** le filtre en charbon actif (21, 31) est adapté à être chauffé à une température comprise entre 40°C et 120°C.

8. Hotte d'aspiration d'un flux d'air, **caractérisée en ce qu'**elle comporte un conduit de circulation (10) d'un flux d'air conforme à l'une des revendications 1 à 7.

## Claims

1. Conduit for circulating an air flow, comprising a device for treating volatile organic compounds or gases in an air flow, comprising suction means (23) and a filter based on active carbon (21, 31), the active-carbon filter (21, 31) being associated with heating means and the suction means (23) being adapted to suck the air flow at at least two different suction rates, said treatment device comprising an auxiliary conduit (26) for circulating the air flow including a photocatalytic material and means forming a light source (24), the filter based on active carbon (21, 31) being disposed upstream of the photocatalytic material activatible by the means forming a light source (24, 32) relative to the direction of circulation of the air flow in the circulation conduit (10), the circulation conduit also comprising means (27) of diverting the air flow comprising at least one flap adapted in a first position to direct the air flow in the circulation conduit (10) and in a second position to direct the air flow solely in the auxiliary conduit (26), said at least one flap (27) being placed in said first position when the suction rate of the suction means (23) is greater than a predetermined threshold value.

2. Circulation conduit according to claim 1, **characterised in that** said at least one flap (27) extends through the circulation conduit (10) and is adapted in said first position to open the circulation conduit (10) and in said second position to close off said circulation conduit (10) apart from said auxiliary conduit (26).

3. Circulation conduit according to claim 2, **characterised in that** said at least one flap (27) is placed in the first position under the effect of the force exerted by the air flow.

4. Circulation conduit according to one of claims 1 to 3, **characterised in that** a first suction rate is between 150 and 1200 m³/h and a second suction rate is between 10 and 50 m³/h.

5. Circulation conduit according to one of claims 1 to 4, **characterised in that** the auxiliary conduit (26) is produced by means of a filter (25) covered with a photocatalytic material.

6. Circulation conduit according to one of claims 1 to 5, **characterised in that** the active-carbon filter (21, 31) is produced as a sheet of active carbon adapted to be electrically connected at two ends so as to heat up by Joule effect.

7. Circulation conduit according to one of claims 1 to 6, **characterised in that** the active-carbon filter (21, 31) is adapted to be heated to a temperature of between 40°C and 120°C.

8. Hood for sucking in an air flow, **characterised in that** it comprises a conduit (10) for circulation of an air flow according to one of claims 1 to 7.

## Patentansprüche

1. Leitung zur Zirkulation eines Luftstroms, mit einer Vorrichtung zur Behandlung von flüchtigen organischen Verbindungen oder Gasen in einem Luftstrom, die Ansaugmittel (23) und einen Filter (21, 31) auf Aktivkohlebasis aufweist, wobei der Aktivkohlefilter (21, 31) Heizmitteln zugeordnet ist und die Ansaugmittel (23) dazu geeignet sind, den Luftstrom mit mindestens zwei unterschiedlichen Ansaugdurchsätzen anzusaugen, wobei die Behandlungsvorrichtung eine Nebenleitung (26) zur Zirkulation des Luftstroms aufweist, in der ein fotokatalytisches Material und eine Lichtquelle (24) bildende Mittel integriert sind, wobei der Filter (21, 31) auf Aktivkohlebasis in Bezug auf die Zirkulationsrichtung des Luftstroms in der Zirkulationsleitung (10) stromaufwärts des fotokatalytischen Materials angeordnet ist, das von den die Lichtquelle (24, 32) bildenden Mitteln aktiviert werden kann, wobei die Zirkulationsleitung ferner Mittel (27) zum Umlenken des Luftstroms mit mindestens einem Ventilelement aufweist, das in einer ersten Stellung den Luftstrom in die Zirkulationsleitung (10) und in einer zweiten Stellung den Luftstrom lediglich in die Nebenleitung (26) lenken kann, wobei sich das mindestens eine Ventilelement (27) in der ersten Stellung befindet, wenn der Ansaugdurchsatz der Ansaugmittel (23) höher ist als ein vorbestimmter Schwellenwert.

2. Zirkulationsleitung nach Anspruch 1, **dadurch gekennzeichnet, dass** sich das mindestens eine Ventilelement (27) durch die Zirkulationsleitung (10) erstreckt und in der ersten Stellung die Zirkulationsleitung (10) öffnen und in der zweiten Stellung die Zirkulationsleitung (10) mit Ausnahme der Nebenleitung (26) verschließen kann.

3. Zirkulationsleitung nach Anspruch 2, **dadurch gekennzeichnet, dass** das mindestens eine Ventilelement (27) unter der Wirkung der von dem Luftstrom ausgeübten Kraft in die erste Stellung gestellt wird.

4. Zirkulationsleitung nach einem der Ansprüche 1 bis 3, **dadurch gekennzeichnet, dass** ein erster Ansaugdurchsatz zwischen 150 und 1200 m³/h und ein zweiter Ansaugdurchsatz zwischen 10 und 50 m³/h beträgt.

5. Zirkulationsleitung nach einem der Ansprüche 1 bis 4, **dadurch gekennzeichnet, dass** die Nebenleitung (26) mit einem Filter (25) gebildet ist, der mit einem fotokatalytischen Material bedeckt ist.

6. Zirkulationsleitung nach einem der Ansprüche 1 bis 5, **dadurch gekennzeichnet, dass** der Aktivkohlefilter (21, 31) aus einem Aktivkohlegewebe besteht, das an zwei Enden elektrisch angeschlossen werden kann, so dass es sich durch Joule-Effekt erwärmt.

7. Zirkulationsleitung nach einem der Ansprüche 1 bis 6, **dadurch gekennzeichnet, dass** der Aktivkohlefilter (21, 31) auf eine Temperatur zwischen 40°C und 120°C erwärmt werden kann.

8. Haube zum Abziehen eines Luftstroms, **dadurch gekennzeichnet, dass** sie eine Leitung (10) zur Zirkulation eines Luftstroms nach einem der Ansprüche 1 bis 7 aufweist.
